# EUROPÄISCHE PATENTSCHRIFT

(11) **EP 1 427 430 B1**
(45) Veröffentlichungstag und Bekanntmachung des Hinweises auf die Patenterteilung: **27.04.2005**
(21) Anmeldenummer: 02798728.8
(22) Anmeldetag: 16.09.2002
(51) Int. Cl.: A61K 35/78, A61P 13/00, A61P 15/00

(54) **VERWENDUNG VON EXTRAKTEN AUS CIMICIFUGA-ARTEN ALS ORGANSELEKTIVES ARZNEIMITTEL ZUR BEHANDLUNG VON SEXUALHORMONABHÄNGIGEN ERKRANKUNGEN DES UROGENITALTRAKTES**
USE OF EXTRACTS OF THE GENUS CIMICIFUGA AS ORGANOSELECTIVE MEDICINES FOR TREATING DISEASES OF THE GENITOURINARY SYSTEM CAUSED BY SEX HORMONES
UTILISATION D'EXTRAITS DE L'ESPECE CIMICIFUGA EN TANT QUE MEDICAMENTS ORGANOSELECTIFS POUR TRAITER DES MALADIES DE L'APPAREIL GENITO-URINAIRE CAUSEES PAR DES HORMONES SEXUELLES

(30) Priorität: 19.09.2001 DE 10146159
(43) Veröffentlichungstag der Anmeldung: 16.06.2004
(73) Patentinhaber: Bionorica AG, 92318 Neumarkt (DE)
(72) Erfinder: WUTTKE, Wolfgang, 37120 Bovenden (DE); JARRY, Hubertus, 37249 Neu-Eichenberg (DE); CHRISTOFFEL, Volker, 92369 Buchberg (DE); SPENGLER, Barbara, 92318 Neumarkt (DE)
(74) Vertreter: Kaiser, Jürgen, Dr.rer.nat.Dipl.-Chem.
(86) Internationale Anmeldenummer: PCT/EP2002/010383
(87) Internationale Veröffentlichungsnummer: WO 2003/024465

(56) Entgegenhaltungen:
- WO-A-02/36140
- WO-A-99/47149
- LIU J ET AL: "EVALUATION OF ESTROGENIC ACTIVITY OF PLANT EXTRACTS FOR THE POTENTIAL TREATMENT OF MENOPAUSAL SYMPTOMS" JOURNAL OF AGRICULTURAL AND FOOD CHEMISTRY, AMERICAN CHEMICAL SOCIETY. WASHINGTON, US, Bd. 49, Nr. 5, 2001, Seiten 2472-2479, XP001068359 ISSN: 0021-8561
- BORRELLI FRANCESCA ET AL: "Cimicifuga racemosa: A systematic review of its clinical efficacy." EUROPEAN JOURNAL OF CLINICAL PHARMACOLOGY, Bd. 58, Nr. 4, Juli 2002 (2002-07), Seiten 235-241, XP002237716 ISSN: 0031-6970

## Beschreibung

Die vorliegende Erfindung betrifft eine Verwendung von Extrakten aus Cimicifuga-Arten gemäß Anspruch 1 und 3.

Aus WO99/47 149 (= EP1064009A1) der Anmelderin der vorliegenden Anmeldung ist bekannt, daß Extrakte aus Cimicifuga racemosa keine östrogene Wirkung am Uterus, wohl aber in der hypothalamo-hypophysären Achse, im Herz-Kreislauf-System und im Knochen ausüben. Insoweit wurden Extrakte aus Cimicifuga racemosa verwendet, um ein estrogenartiges organselektives Arzneimittel ohne uterotrope Wirkung herzustellen.

Lui J. et al, Journal of Agricultural and Food Chemistry, American Chemical Society, Washington, US, Bd. 49, Nr. 5, 2001, S.2472-2479 beschreibt, dass Extrakte von Cimicifuga racemosa für die Behandlung von Wechseljahresbeschwerden, wie Hikewallungen, Depressionen, Stimmungsschwankungen, Schlafsförungen, trockene Vagina und Gelenkschmerzen eingesetzt werden.

WO 02/36140 beschreibt, dass Zubereitungen von Cimicifuga racemosa beim Vorliegen einer Harninkontinenz therapeutisch wirksam sind.

Das bei Frauen in den Ovarien gebildete 17β-Estradiol (wenn im folgenden von Estradiol gesprochen wird, ist immer das physiologische wirksame 17β-Estradiol gemeint) [im folgenden auch mit E₂ bezeichnet] hat im Organismus eine allgemein proliferationsfördemde Wirkung. Neben der Steuerung des Zyklus der Frau besitzt es u.a. einen homöostatischen Einfluss auf die Funktion des Harnleiters, der Harnblase und der Vagina. Bei Männern wirkt lokal gebildetes Estradiol sowie das männliche Geschlechtshormon Testosteron ebenfalls in der Harnblase und in der Prostata proliferationsfördernd.

In der Menopause kommt es zu einem Absinken der Estradiolspiegel in Folge des Erlöschens der Ovarialfunktion. Daraus resultiert eine Abschwächung proliferativer Prozesse in vielen Organen in deren Folge Degeneration und funktionelle Minderleistungen auftreten.

Unter Estrogenmangel entwickelt sich bei der postmenopausalen Frau häufig eine Blasenentzündung als Folge reduzierter Schleimhaut in der Harnröhre und damit erleichterter Keimaszension.

Auch die postmenopausal häufig auftretende Harninkontinenz wird durch Estrogenmangel verursacht. Diese Erkrankungen können durch rechtzeitige Estradiolgabe verhindert, bzw. gebessert werden.

Die Funktion der Harnblase ist wahrscheinlich bei Männern hormonell ähnlich reguliert wie bei Frauen; jedenfalls sind die bisher bekannten regulativen Prinzipien in der Harnblase weiblicher und männlicher Ratten sehr ähnlich.

Die Gabe von Estrogenen mit unselektiver proliferativer Wirkung ist insbesondere für Uterus und Brustgewebe nachteilig, da es hier durch die unzureichende oder fehlende Gestagen-Gegenregulation zu einem unkontrollierten Wachstum kommen kann.

Bislang steht kein Arzneimittel aus pflanzlichen Drogen zur Verfügung , welches zu einer organselektiven Prophylaxe oder Therapie von Veränderungen im Urogenitaltrakt bei Estrogenmangel verwendet werden kann.

Ausgehend von diesem Stand der Technik ist es daher Aufgabe der vorliegenden Erfindung, pflanzliche Arzneimittel mit sexualhormonartiger Wirkung zur Verfügung zu stellen, welche eine organselektive Wirkung ohne oder mit nur geringer Wirkung auf den Uterus aufweisen.

Die Lösung dieser Aufgabe erfolgt durch die kennzeichnenden Merkmale der Ansprüche 1 und 4.

Insbesondere betrifft die vorliegende Erfindung eine
Verwendung von Extrakten aus Cimicifuga-Arten, insbesondere Cimicifuga racemosa, zur Herstellung eines estrogenartigen organselektiven Arzneimittels ohne oder wenigstens mit zu vernachlässigender uterotroper Wirkung, wobei
das Arzneimittel zur Linderung von sexualhormonbedingten Erkrankungen des Urogenitaltraktes verwendet wird.
Bevorzugt ist eine Verwendung bei postmenopausal auftretender Harnblasenentzündung.
Darüber hinaus betrifft die vorliegende Erfindung eine Verwendung von Extrakten aus Cimicifuga-Arten, insbesondere Cimicifuga racemosa, zur Herstellung eines estrogenartigen organselektiven Arzneimittels ohne oder wenigstens mit zu vernachlässigender uterotroper Wirkung, wobei
das Arzneimittel zur Linderung der benignen und malignen Hyperplasie der Prostata eingesetzt wird.

Überraschenderweise hat sich gezeigt, daß Cimicifuga racemosa-Extrakt, beispielsweise aus den Rhizomen von Cimicifuga racemosa, auch in der Harnblase und im Harnleiter ovarektomierter Ratten ähnliche Effekte wie Estradiol ausübt. In der Blase werden sowohl der Estrogenrezeptor ERalpha wie auch ER-beta exprimiert. Es wurde gefunden, daß Estradiol und Cimicifuga in gleichsinniger Weise die Genexpression des Estrogenrezeptors-alpha in der Harnblase herunterregulieren. Somit scheint der Blasentonus neben der Innervation auch durch Steroidhormone beeinflussbar zu sein.

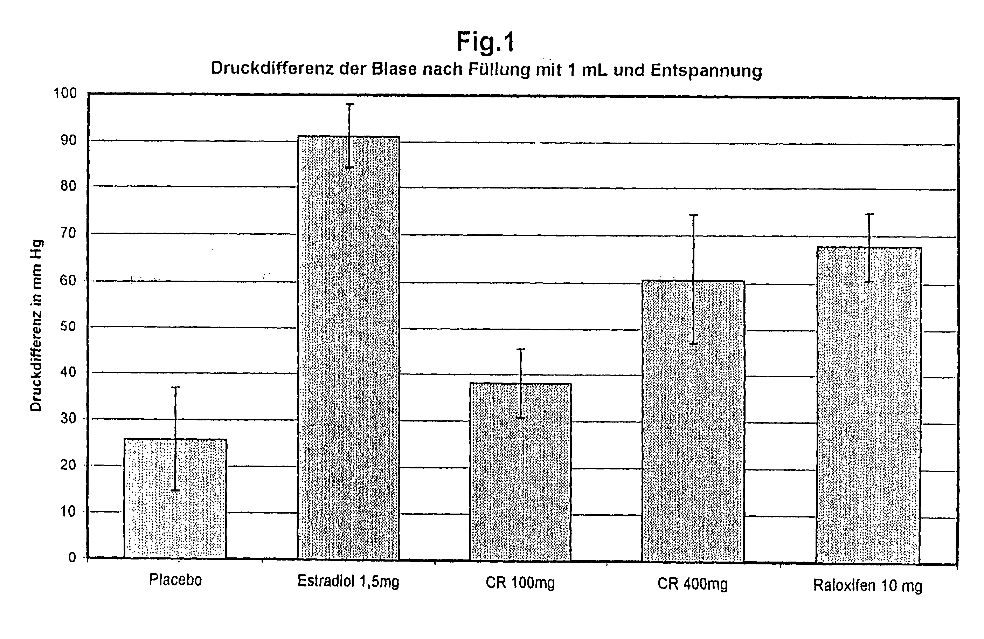

In Fig. 1 ist ein Diagramm gezeigt, welches den Blasendifferenztonus von ovarektomierten Ratten (Gruppengröße 12 Tiere) wiedergibt. Nach einer dreimonatigen Behandlung von ovarektomierten Ratten mit Cimicifuga racemosa-Extrakt wurde durch Instillation von 1 ml physiologischer Kochsalzlösung in die Harnblase der maximale Druck [in mmHg] und der minimale Druck [in mmHg] nach Entspannung der Blase bestimmt.

Das Diagramm gemäß Fig. 1 zeigt auf der Ordinate den Differenzdruck [in mmHg] zwischen maximalem und minimalem Druck an, der ein Maß für die Elastizität der Harnblase ist.

Es zeigt sich im Vergleich zu Placebokontrollen nach Behandlung mit Cimicifuga-Extrakt eine signifikante dosisabhängige Erhöhung der Blasenelastizität, gemessen anhand der Druckdifferenz zwischen entspannter und definiert gefüllter Blase.

In dem Testsystem erweist sich Estradiol als die wirksamste Substanz. Die höchste Cimicifuga-Dosis (400 mg/kg/d) löst eine Wirkung aus, die mit der des synthetischen SERM (Selective Estrogen Receptor Modulator) Raloxifen, vergleichbar ist.

Damit ist ein zusätzlicher Effekt des Extraktes auf die Harnblase, nämlich eine Verbesserung der Blaseninkontinenz, ebenfalls gesichert.

Die benigne Prostata-Hyperplasie und das Prostata-Karzinom des Mannes können durch Hemmung der lokalen Estrogen-Produktion, aber auch durch Hemmung der Synthese der männlichen Geschlechtshormone ebenfalls günstig beeinflußt werden.

Derartige Wirkmechanismen sind auch an der kastrierten männlichen Ratte für den Cimicifuga racemosa-Extrakt nachweisbar. Bei Versuchen der Erfinder der vorliegenden Anmeldung hat sich herausgestellt, daß Estradiol und der Cimicifuga-Extrakt in der Prostata signifikant die Genexpression des Estrogenrezeptors vom alpha-Subtyp inhibieren, ohne einen Effekt auf die Genexpression des Estrogenrezeptor-beta auszuüben.

Sowohl bei in vivo Experimenten in der Prostata der Ratte im Ganzkörpermodell als auch in humanen Prostata-Karzinomzellen hat sich überraschend herausgestellt, daß Cimicifuga racemosa-Extrakte einen antiandrogenen Effekt ausüben, die das Prostatakarzinom günstig beeinflussen und das Wachstum der Tumorzellen in vitro signifikant hemmen.

In der Prostata kastrierter Rattenmännchen wird femer die Genexpression des proliferationsfördernden Wachstumsfaktors IGF1 durch Testosteron hochreguliert, durch Cimicifuga jedoch herunterreguliert, wie es für eine Reduktion der Hyperplasie erwünscht ist. Die durch Kastration induzierte Prostataregression konnte experimentell in der Ratte durch Testosteron antagonisiert werden. In Analogie zu den Befunden an kastrierten, nicht mit Androgen behandelten Ratten (siehe oben) konnte auch die durch Testosteron hochregulierte IGF1 Expression (siehe oben) durch den Cimicifuga-Extrakt herunterreguliert werden. Diese Befunde belegen eindeutig, daß das Testosteron-induzierte Prostata-Wachstum durch Extrakte aus Cimicifuga-Arten inhibiert werden kann.

In Experimenten an der humanen Prostata-Ca-Linie LNCAP ist die 5 alpha-Dihydrotestosteron - induzierte Proliferation und die Produktion von deren prostataspezifischem Antigen (PSA) durch Extrakte aus Cimicifuga-Arten, insbesondere Cimicifuga racemosa, signifikant inhibierbar.

Da IGF1 immer an der Proliferation nicht nur benigner sondern auch maligner Tumoren beteiligt ist, belegt die inhibitorische Wirkung von Extrakten aus Cimicifuga-Arten auf die IGF1-Genexpression zusammen mit diesem Befund eine hemmende Wirkung auf das Prostata-Karzinom.

Zur Herstellung des erfindungsgemäßen Extraktes wird vollinhaltlich auf die WO99/47 149 verwiesen, deren wesentlichen Merkmale jedoch im folgenden zusammengefaßt sind:

Bei in vitro und in vivo Versuchen hat sich herausgestellt, dass aus Cimicifuga-Arten, insbesondere Cimicifuga racemosa, mit organischen Lösungsmitteln oder Gemischen von organischen Lösungsmitteln mit Wasser oder mit überkritischem CO₂ hergestellte Extrakte organselektiv auf das urogenitale System wirken, wobei eine Wirkung auf den Uterus fehlt.

Daher sind die erfindungsgemäß verwendeten Extrakte zur Herstellung eines fertig formulierten Mittels zur selektiven Behandlung und/oder Prophylaxe von Sexualhormon-bedingten Erkrankungen des Urogenitaltraktes geeignet.

Desweiteren sind sie zur Herstellung eines fertig formulierten Mittels zur selektiven Behandlung und/oder Prophylaxe postmenopausaler Harnblaseninfektionen geeignet.

Sie sind ferner zur Herstellung eines fertig formulierten Mittels zur Behandlung von benignen und malignen Prostatahyperplasien geeignet.

Es wurde weiterhin gefunden, dass angereicherte und/oder reine Inhaltsstoffe bzw. Gruppen von Inhaltsstoffen aus Cimicifuga-Arten im wesentlichen die gleichen Wirkungen ausüben wie der Gesamtextrakt.

Als Inhaltsstoffe bzw. Inhaltsstoffgruppen kommen insbesondere die folgenden in Betracht: Triterpene beispielsweise Acteol- und Cimicigenolderivate; Polyphenole, wie Kaffeesäure und deren Derivate, Chlorogensäure, Piscidinsäure, Fucinolsäure, Ferula- und Isoferulasäure, Cimicifuginsäuren; Formononetin; Alkaloide beispielsweise Cytisin und Methylcytisin.

Diese Inhaltsstoffe werden neben der Cimicifuga racemosa auch in weiteren Cimicifuga-Arten gefunden, wie z.B. C. foetida, C. dahurica, C. heracleifolia, C. simplex, C. japonica, C. europaea, C. acerina, C. elata, C. rubifolia.

Diese weiteren Cimicifuga-Arten können somit ebenfalls zur Herstellung von Extrakten und damit Medikamenten gegen die im Zusammenhang mit der vorliegenden Erfindung genannten Sexualhormon-bedingten Erkrankungen des Urogenitaltraktes verwendet werden.

Bevorzugt werden zur Herstellung der Extrakte Rhizome, Wurzeln, Stengel, Blätter und/oder Blütenblätter der Pflanzen verwendet.

## Patentansprüche

1. Verwendung von Extrakten aus Cimicifuga-Arten zur Herstellung eines estrogenartigen organselektiven Arzneimittels ohne oder wenigstens mit zu vernachlässigender uterotroper Wirkung,
**dadurch gekennzeichnet, daß**
das Arzneimittel zur Linderung von Sexualhormon-bedingter postmenopausaler Harnblasenentzündung verwendet wird.

2. Verwendung von Extrakten aus Cimicifuga-Arten zur Herstellung eines estrogenartigen organselektiven Arzneimittels,
**dadurch gekennzeichnet, daß**
das Arzneimittel zur Linderung der benignen und malignen Hyperplasie der Prostata eingesetzt wird.

3. Verwendung nach einem der Ansprüche 1 oder 3, **dadurch gekennzeichnet, daß** die Cimicifuga-Art ausgewählt wird aus der Gruppe bestehend aus:
C. racemosa, C. foetida, C. dahurica, C. heracleifolia, C. simplex, C. japonica, C. europaea, C. acerina, C. elata, C. rubifolia.

4. Verwendung nach einem der Ansprüche 1 bis 3, **dadurch gekennzeichnet, daß** Cimicifuga racemosa als Cimicifuga-Art eingesetzt wird.

## Claims

1. Use of extracts from *Cimicifuga* species for producing an estrogen-type organoselective medicament having no uterotrophic effect or one that is at least negligible,
**characterized in that**
the medicament is utilized for alleviating sexual hormone-related postmenopausal urinary bladder infection.

2. Use of extracts from *Cimicifuga* species for producing an estrogen-type organoselective medicament,
**characterized in that**
the medicament is utilized for alleviating benign and malignant prostate hyperplasy.

3. Use in accordance with any one of Claims 1 or 3, **characterized in that** the *Cimicifuga* species is selected from the group consisting of:
C. racemosa, *C. foetida, C. dahurica, C. heracleifolia, C. simplex, C. japonica, C. europaea, C. acerina, C. elata, C. rubifolia*.

4. Use in accordance with any one of Claims 1 to 3, **characterized in that** *Cimicifuga racemosa* is utilized as the *Cimicifuga* species.

## Revendications

1. Utilisation d'extraits de variétés de *Cimicifuga* pour préparer un médicament organosélectif de type oestrogène dépourvu d'effet utérotrope ou du moins possédant un effet utérotrope négligeable,
**caractérisée en ce que**
le médicament est utilisé pour soulager l'inflammation post-ménopausique de la vessie provoquée par les hormones sexuelles.

2. Utilisation d'extraits de variétés de *Cimicifuga* pour préparer un médicament organosélectif de type oestrogène,
**caractérisée en ce que**
le médicament est utilisé pour soulager l'hyperplasie bénigne et maligne de la prostate.

3. Utilisation selon l'une des revendications 1 ou 2, **caractérisée en ce que** la variété de *Cimicifuga* est choisie dans le groupe constitué par
*C. racemosa, C. foetida, C. dahurica, C. heracleifolia, C. simplex, C. japonica, C. europaea, C. acerina, C. elata, C. rubifolia*.

4. Utilisation selon l'une des revendications 1 à 3, **caractérisée en ce que** la variété de *Cimicifuga* utilisée est *Cimicifuga racemosa*.
